Europäisches Patentamt

European Patent Office

Office européen des brevets

(19)

(11) Veröffentlichungsnummer: **0 261 554 B1**

# EUROPÄISCHE PATENTSCHRIFT

(12)

(45) Veröffentlichungstag der Patentschrift: **21.08.91**

(51) Int. Cl.⁵: **C07C 31/20**, C07C 29/86

(21) Anmeldenummer: **87113453.2**

(22) Anmeldetag: **15.09.87**

(54) **Verfahren zur Reinigung von Propandiol-1,3.**

(30) Priorität: **24.09.86 DE 3632397**

(43) Veröffentlichungstag der Anmeldung:
**30.03.88 Patentblatt 88/13**

(45) Bekanntmachung des Hinweises auf die
Patenterteilung:
**21.08.91 Patentblatt 91/34**

(84) Benannte Vertragsstaaten:
**BE DE FR GB NL**

(56) Entgegenhaltungen:

**Keine**

(73) Patentinhaber: **HOECHST AKTIENGESELL-
SCHAFT
Postfach 80 03 20
W-6230 Frankfurt am Main 80(DE)**

(72) Erfinder: **Breitkopf, Norbert
Heinrichstrasse 10
W-4200 Oberhausen 12(DE)**
Erfinder: **Dämbkes, Georg, Dr. Dipl.-Chem.
Nibelungenstrasse 65
W-4220 Dinslaken(DE)**
Erfinder: **Bach, Hanswilhelm, Dr. Dipl.-Chem.
Alleestrasse 56
W-4100 Duisburg 11(DE)**

## Beschreibung

Die Erfindung betrifft ein Verfahren zur Reinigung von Propandiol, das durch Anlagerung von Wasser an Acrolein und Hydrierung des als Primärprodukt entstandenen 3-Hydroxypropanals erhalten wird. Zur Entfernung störender Nebenprodukte behandelt man das Diol erfindungsgemäß mit Cyclohexan.

Propandiol-1,3 ist ein wertvolles Zwischenprodukt, das insbesondere auf dem Gebiet der Lösungsmittel und der Kunststoffe Anwendung findet. Seine Herstellung im technischen Maßstab erfolgt vorwiegend durch katalytische Anlagerung von Wasser an Acrolein (vgl. US-PS 24 34 110). Hierzuwird eine wäßrige Lösung von Acrolein mit soviel Säure versetzt, daß sich ein pH-Wert von etwa 0,5 bis etwa 7 einstellt. In Gegenwart eines Antioxidans, z.B. Hydrochinon, erhitzt man 0,5 bis 8 Stunden auf Temperaturen, die 100°C nicht übersteigen. Anschließend wird das Reaktionsgemisch in Gegenwart von Raney-Nickel hydriert. Varianten oder Weiterentwicklungen dieses Verfahrens betreffen die Anwendung von Ionenaustauschern als Katalysatoren für die Wasseranlagerung (vgl. US-PS 35 36 763) und die Hydrierung des 3-Hydroxypropanals in einem organischen Lösungsmittel, z.B. Isobutanol, in Gegenwart von Nickelträgerkatalysatoren (vgl. DE-PS 20 54 601). Aus dem Hydrierprodukt erhält man Propandiol-1,3 durch mehrstufige Destillation in einer Reinheit von mehr als 99 %.

Auch nach sorgfältiger Destillation können in reinem Propandiol-1,3 Trübungen auftreten. Sie stehen im Zusammenhang mit der Qualität des eingesetzten Acroleins. Durch Diensynthese kann nämlich aus zwei Molekülen Acrolein 2-Formyl-2,3-dihydro-gamma-pyran(dimeres Acrolein) entstehen, das mit Propandiol-1,3 unter Bildung des Halbacetals und Acetals reagiert. Diese Aldehydderivate sind Ursache der Trübungen. In einigen Fällen beeinträchtigen sie die Weiterverarbeitung des Diols in erheblichem Maße.

Es bestand daher die Aufgabe, ein Verfahren zu entwickeln, das Trübungen verursachende Verunreinigungen aus dem Diol zurverlässig entfernt.

Die Erfindung besteht in einem Verfahren zu Reinigung von Propandiol-1,3, das durch Anlagerung von Wasser an Acrolein erhalten wurde. Es ist dadurch gekennzeichnet, daß das Diol mit Cyclohexan extrahiert wird.

Mit Hilfe der neuen Arbeitsweise gelingt es, reines Propandiol-1,3 zu erhalten, das frei von Trübungen ist und höchsten Anforderungen an die Reinheit genügt.

Es war nicht zu erwarten, daß die Reinigung des Diols durch einfache Extraktion in einem ausgewählten Lösungsmitel zu hervorragenden Ergebnissen führt. Versuche, durch Feindestillation das Problem zu lösen, blieben ebenso erfolglos, wie Versuche zu Extraktion mit den verschiedensten aliphatischen, cycloaliphatischen und aromatischen Kohlenwasserstoffen unter wechselnden Bedingungen. Überraschenderweise erwies sich Cyclohexan als vorzüglich geeignetes Extraktionsmittel. Besonders hervorzuheben ist, daß sich die beiden Phasen Diol und Kohlenwasserstoff nach der Extraktion sehr leicht voneinander trennen und daß Spuren des Extraktionsmittels, die im Extraktionsgut zurückbleiben, einfach durch Abdampfen entfernt werden können. Die Löslichkeit von Propandiol-1,3 im Cyclohexan ist so gering, daß Diolverluste kaum auftreten.

In der Praxis erfolgt die Reinigung des Propandiols-1,3 mit Cyclohexan durch Gegenstromextraktion, wobei im allgemeinen eine einzige Extraktionsstufe ausreicht. In seltenen Fällen kann es erforderlich sein, mehrstufig durch Gegenstrom- oder Kreuzstromextraktion zu extrahieren. Je 100 Gewichtsteile Diol setzt man etwa 2 bis 10 und insbesondere 4 bis 6 Gewichtsteile Extraktionsmittel ein. Die Abtrennung der Verunreinigungen aus dem Diol wird bei 20 bis 60°C und insbesondere bei 30 bis 50°C durchgeführt. Nach der Reinigung im Diol enthaltenes Cyclohexan wird durch Abdampfen zweckmäßig im Vakuum bei 50 bis 150 hPa entfernt.

Das neue Verfahren liefert qualitativ einwandfreies Propandiol-1,3, das klar und geruchsneutral ist.

Die neue Arbeitsweise wird durch die nachfolgenden Beispiele näher erläutert.

### Beispiel 1

100 g trübes Propandiol-1,3 werden in einem Rührkolben bei Raumtemperatur mit 5 g Cyclohexan versetzt und intensiv gemischt. Nach Trennung der Phasen, die mehr als eine Stunde erfordert, erhält man eine klare Diolphase. Sie wird bei 100°C im Vakuum (etwa 100 hPa) vom restlichen Cyclohexan befreit. Es bleiben 99 g wasserklares und geruchsfreies Propandiol-1,3 zurück.

### Beispiel 2

Der in Beispiel 1 beschriebene Versuch wird wiederholt, die Arbeitstemperatur aber auf 50°C erhöht. Unter diesen Bedingungen erfolgt die Phasentrennung innerhalb weniger Minuten. Nach der Aufarbeitung wie in Beispiel 1 bleiben 99 g wasserklares und geruchsfreies Produkt zurück.

Beispiele 3 und 4 - zum vergleich

Beispiel 1 wird unter Verwendung von i-Octan und Toluol als Extraktionsmittel wiederholt. Die Ergebnisse sind nachfolgend dargestellt.

| Beispiel Nr. | 3 | 4 |
|---|---|---|
| Extraktionsmittel | i-Octan | Toluol |
| Propandiol-Ausbeute | 98% | 99% |
| Propandiol-Qualität | trübe | trübe |

Beispiele 5 und 6 - zum vergleich

Die Beispiele 3 und 4 werden bei auf 50°C erhöhter Arbeitstemperatur wiederholt. Die Ergebnisse sind nachfolgend dargestellt.

| Beispiel Nr. | 5 | 6 |
|---|---|---|
| Extraktionsmittel | i-Octan | Toluol |
| Propandiol-Ausbeute | 99% | 99% |
| Propandiol-Qualität | trübe | trübe |

**Patentansprüche**

1. Verfahren zur Reinigung von Propandiol-1,3, das durch Anlagerung von Wasser an Acrolein erhalten wurde, dadurch gekennzeichnet, daß das Diol mit Cyclohexan extrahiert wird.

2. Verfahren nach Anspruch 1, dadurch gekennzeichnet, daß zur Extraktion je 100 Gewichtsteile Diol 2 bis 10, insbesondere 4 bis 6 Gewichtsteile Cyclohexan eingesetzt werden.

3. Verfahren nach einem oder mehreren der vorangegangenen Ansprüche, dadurch gekennzeichnet, daß die Extraktion bei 20 bis 60°C durchgeführt wird.

**Claims**

1.  A process for purifying propanediol-1,3 obtained by hydrating acrolein with water, characterised in that the diol is extracted with cyclohexane.

2.  A process according to claim 1, characterised in that 2 to 10, in particular 4 to 6 parts by weight of cyclohexane are used for extraction per 100 parts by weight of diol.

3.  A process according to one or both of the above claims, characterised in that extraction is carried out at 20 to 60° C.

**Revendications**

1.  Procédé pour la purification du propanediol-1,3 qui a été obtenu par addition d'eau sur l'acroléine, caractérisé en ce que le diol est extrait par le cyclohexane.

2.  Procédé selon la revendication 1, caractérisé en ce que l'on utilise pour l'extraction 2 à 10 parties en poids de cyclohexane par 100 parties en poids de diol, en particulier 4 à 6 parties en poids de cyclohexane.

3.  Procédé selon l'une ou plusieurs des revendications précédentes, caractérisé en ce que l'extraction est mise en oeuvre à 20-60° C.